# EUROPEAN PATENT SPECIFICATION

(11) **EP 2 618 892 B1**
(45) Date of publication and mention of the grant of the patent: **23.05.2018**
(21) Application number: 11773658.7
(22) Date of filing: 20.09.2011
(51) Int. Cl.: A61Q 17/04, A61K 8/26, A61K 8/29, A61K 8/49, A61K 8/58, A61K 8/27

(54) **HIGH PROTECTION UVA/UVB COMPOSITION AND TOPICAL COSMETIC COMPOSITION**
UVA/UVB-ZUSAMMENSETZUNG MIT HOHEM SCHUTZFAKTOR UND TOPISCHE KOSMETISCHE ZUSAMMENSETZUNG
COMPOSITION DE HAUTE PROTECTION CONTRE LES UVA/UVB ET COMPOSITION COSMÉTIQUE TOPIQUE

(30) Priority: 20.09.2010 FR 1057494
(43) Date of publication of application: 31.07.2013
(73) Proprietor: Natura Cosméticos S.A., 06882-700 ltapecerica da Serra - SP (BR)
(72) Inventor: PICONI LONGO, Ana Carolyna, 04522-032 São Paulo, SP (BR); AZEVEDO TADINI, Kassandra, 13210-705 Jundai, SP (BR); COLLINA ROMANHOLE, Rodrigo, 03120-010 Mooca São Paulo - SP (BR)
(74) Representative: Abel & Imray
(86) International application number: PCT/BR2011/000336
(87) International publication number: WO 2012/037627

(56) References cited:
- EP-A1- 0 798 267
- EP-A1- 0 922 740
- EP-A1- 2 092 929
- WO-A2-01/05362
- WO-A2-02/094205
- FR-A1- 2 591 480
- FR-A1- 2 898 055
- US-A1- 2003 144 398
- US-A1- 2007 264 292

## Description

The present invention refers to a topical cosmetic or pharmaceutical high protection UVA / UVB composition comprising mainly physical filters, with high stability and superior sensory characteristics.

### BACKGROUND OF THE INVENTION

It is known that the sun radiation on contact with skin may cause several types of injuries. In order to protect the skin, it is commonly used sunscreen products comprising substances capable of reflecting, scattering or absorbing photons of ultraviolet region (UV).

Among the various types of radiation emitted by the sun, there are the infrared, UVA, UVB and UVC, wherein the UV rays are the main cause of skin damage.

UVC-rays are absorbed by the higher layers of the atmosphere and stratosphere, and rarely reach the ground. With respect to UVA-rays and a portion of UVB-rays, they are transmitted through the atmosphere and, therefore, can damage the skin. As the UVB-rays have shorter wavelengths, they reach the most superficial layer of the skin - the epidermis, while UVA-rays can penetrate to the dermis - the middle layer.

Excessive exposure to the sun can result in skin burns, also known as erythema, caused mainly by UVB radiation. Over the longer term, UV radiation induces degenerative changes in cells of the skin, fibrous tissue and blood vessels leading to premature skin aging, photodermatoses, actinic keratoses and even cancer.

The sun protection factor (SPF) is a factor commonly used in sunscreens to indicate how much the product can protect the skin. SPF is a measure of how much solar energy is required to produce erythema on protected skin (i.e., in the presence of the sunscreen) relative to the amount of solar energy required to produce sunburn on unprotected skin. As the SPF value increases, sunburn protection increases. The SPF factor only indicates protection against UVB-rays, responsible for skin burns. For UVA-rays, ways of measuring and protection indicators are currently still being studied.

One of the most widely known methods to determine the protective factor for UVA is the Persistent Pigment Darkening (PPD). According to this method, the immediate pigmentation resulted from photooxidation of preexisting melanin occurring caused by UVA radiation exposure is measured. According to Colipa (The European Cosmetic Association), the recommended PPD for sunscreen products should be at least one third of the SPF.

Products for protection against the harmful rays of the sun comprise sunscreens and, according to their mechanism of action, may be classified as:
- physical filters → substances capable of reflecting and scattering UV rays, and
- chemical filters → substances capable of absorbing UV radiation and convert it into radiation with lower energy and less harmful to humans.

The damage associated with sun exposure has a cumulative effect, which contributes to the emergence of cancer many years after the exposure. Recent studies show that sun protection in childhood and adolescence significantly reduces the risk of skin cancer. About 80% of all solar radiation received over a lifetime is concentrated in the first 18 years of age.

Particularly for babies, it is recommended the use of sunscreens containing physical filters (titanium dioxide and zinc oxide) since the skin of babies is more sensitive than the skin of adults. These filters are considered less irritating and effective blockers of solar radiation. It is desirable that the sunscreen products for babies have a high SPF, an appropriate PPD and broad spectrum.

Compositions with high SPF and high UVA protection usually contain a large amount of sun filters. However, the high load of filters generally affect the sensory and stability characteristics of the composition.

Particularly, high-protection compositions comprising only physical filters tend to have the following disadvantages:
- sticky aspect and sensory;
- low spreadability;
- whitish appearance after application;
- preparation of the formulation is difficult due to the high insolubility of the filters, and
- instability of the composition.

Generally, in order to increase the SPF of a sunscreen composition comprising physical filters, the incorporation of chemical filters is used.

The document WO03072077 discloses a sunscreen composition comprising physical solar filters and which is transparent and clear when applied to the skin. However, the maximum FPS of this composition is equal to 41.2.

The document WO9852525 describes a transparent sunscreen composition containing zinc oxide. However, unlike the composition of the present invention, the composition described in this document also contains chemical sunscreens.

The document PI9006393 discloses a transparent composition containing inorganic sunscreens such as titanium dioxide and zinc oxide. Nevertheless, the composition of this document does not have a high SPF. The document FR2591480 describes sunscreen compositions based on inorganic filters including zinc oxide and titanium dioxide.

Hence, the state of the art does not reveal a high protection UVA / UVB sunscreen composition with a high SPF and an appropriated PPD comprising mainly physical filters, having pleasant sensory characteristics, no stickiness, good spreadability, transparency after applying and high resistance to water after be applied to the skin as well as ease of preparation and high stability in storage.

Because of the exclusive presence of physical filters, the composition described in the present application is particularly suitable for babies and people who have some type of dermal irritation when using chemical sunscreens.

### SUMMARY OF THE INVENTION

The present invention relates to a topical cosmetic or pharmaceutical high protection UVA / UVB composition comprising (a) physical filter selected from the group comprising titanium dioxide, zinc oxide, a benzotriazole-based compound and mixtures thereof, and (b) a non-animal hectorite dispersion.

The composition of the present invention has the following advantages in relation to the state of art:
- sun protection with a broad-spectrum (UVA / UVB), high SPF and appropriated PPD;
- high stability;
- it is transparent after application;
- high water resistance;
- good spreadability
- nice sensory characteristics;
- no stickiness; and
- particularly suitable for babies and people with sensitive skin, since it comprises mainly physical filters.

A further advantage of the high protection UVA / UVB composition as claimed is that it is not necessary the presence of film former, since the physical filters has an improved skin adhesion, hence the present composition is more resistance to water and sweat.

### BRIEF DESCRIPTION OF THE DRAWINGS

Figure 1 shows the kinetic degradation of the high protection UVA / UVB composition of the present invention without irradiation thereof, and after 1, 2, 4 and 8 hours of exposure equivalent to natural sunlight.
Figure 2 shows the percentage decreasing in the UVA (320 to 400nm) protection for the high protection UVA / UVB protection composition of the present invention.
Figure 3 shows the graphics generated for the high protection UVA / UVB protection composition of the present invention before and after exposure to radiation in the PMMA plate.

### DETAILED DESCRIPTION OF THE INVENTION

The present invention relates to a topical cosmetic or pharmaceutical high protection UVA / UVB composition comprising:
(a) 24 to 45.5 % by weight of physical filter selected from the group comprising titanium dioxide particles, zinc oxide particles, a benzotriazole-based compound particles and mixtures thereof;
(b) 6 to 10% by weight of a non-animal hectorite dispersion, and
(c) optionally, 2 to 20% by weight of a sensory modifier selected from the group comprising: silicones, esters, silica and mixtures thereof.
wherein all percentages are based on the total weight of the high protection UVA / UVB composition.

The high protection UVA / UVB composition of the present invention is a cosmetic and pharmaceutical composition for topical use.

In a more preferred embodiment of the present invention, the content of physical filters is 34% by weight based on the total weight of the composition. Still, the aforementioned composition can achieve a SPF greater than 50 and a PPD of at least 16 without the inconvenience of high SPF compositions already know in the state of art, for example, sticky, whitish appearance in the skin after the application and the difficulty of spreading.

### Physical Filters

The titanium dioxide particles present in the high protection UVA / UVB composition of the present invention are used in the pure form or after a surface treatment, which may be coated with aluminum oxide and aluminum stearate. Furthermore, said titanium dioxide particles are dispersed in a cosmetically acceptable carrier, preferably a lipid carrier.

According to a preferred embodiment of the invention, the dispersion of titanium dioxide particles is present in said high UVA / UVB protection composition in an amount ranging from 5 to 15%, by weight, based on total weight of the composition, preferably 7,5 to 12,5%, even more preferably 10%.

The zinc oxide particles present in the high protection UVA / UVB composition of the present invention are dispersed in a cosmetically acceptable carrier, preferably a lipid carrier.

The dispersion of zinc oxide particles is present in said high protection UVA / UVB composition, preferably in an amount ranging from 10 to 30% by weight, based on total weight of the composition, preferably 15 to 25 %, more preferably 20%.

The benzotriazole-based compound combines the proprieties of a physical filter (reflects the solar radiation) and a chemical filter (absorbs solar radiation). Preferably, this compound is present in the high protection UVA / UVB composition of the present invention in the form of a dispersion.

The dispersion of benzotriazole-based compound is advantageously present in said high protection UVA / UVB composition, in an amount ranging from 1 to 10% by weight, based on total weight of the composition, preferably 2 to 8%, more preferably 4%.

In a preferred embodiment of the present invention, a dispersion of particles of a benzotriazole-based compound used to achieve the objectives of the present invention is Tinosorb M™ (methylene bis-benzotriazolyl tetramethylphenol), produced and marketed by Ciba™.

### Dispersion of Non-Animal Hectorite

The non-animal hectorite dispersion used in the present invention is known to confer rheology control and suspension for the organic based cosmetics or silicone cosmetics.

The composition disclosed in this invention is not organic based nor based on silicone. However, surprisingly, the dispersion used in the present invention confers high stability to the composition, keeping the inorganic agents of the high protection UVA / UVB composition, namely titanium dioxide particles and zinc oxide particles, well dispersed in the emulsion.

The dispersion of non-animal hectorite is present in said high protection UVA / UVB composition in an amount ranging from 6 to 10% by weight, based on total weight of the composition, more preferably 7 to 9%, still more preferably 8%.

In a preferred embodiment of the present invention, the non-animal hectorite dispersion is organically modified with caprylic/capric triglyceride.

### Sensorial Modifier

The sensory modifiers optionally used in the high protection UVA / UVB composition of the present invention can be selected from the group consisting of: silicones, esters of short chain, silica and mixtures thereof.

Preferably, the sensory modifier of the present invention is selected from the group comprising: dimethicone, caprylyl methicone, cyclomethicone, tocopheryl acetate, polysiloxane and mixtures thereof. More preferably, the sensory modifiers used in this invention are selected among cyclomethicone, caprylyl methicone and mixtures thereof.

The sensory modifier may be used in the present composition in an amount ranging from 2 to 20% by weight, based on total weight of the composition, preferably 5 to 15%, even more preferably 10%.

### Other Optional Components

The high protection UVA / UVB composition of the present invention further comprises optional components commonly used in cosmetics or pharmaceuticals in accordance with the intended application for the composition being prepared.

Among the optional components, it can be cited: antioxidant, preservative, film former, support microcrystalline network former, polymeric agent, copolymeric agent, cosolvent, denaturing agent, consistency agent, emollient, humectant, moisturizing agent, constraint agent and mixture thereof.

### EXAMPLE

The present invention will be illustrated through an example in order to better describe it and demonstrate the effectiveness of the high protection UVA / UVB composition described in this patent application. However, this example is merely illustrative of a preferred embodiment of the present invention, therefore, should not be taken as limiting the scope of this patent application.

The high protection UVA / UVB composition comprising the components indicated in Table 1 were submitted to the following tests:
**(1)** Evaluation of skin acceptability in children;
**(2)** Evaluation of skin and eye acceptability in adults;
**(3)** Evaluation of comedogenic potential;
**(4)** Evaluation of photoallergy and phototoxicity in the skin;
**(5)** Evaluation of irritant and allergen potential;
**(6)** Evaluation of skin tolerance;
**(7)** Evaluation of photostability;
**(8)** Evaluation of UVA protection;
**(9)** Evaluation of sun protection factor;
**(10)** Evaluation of stability.

**Table 1: Components of the high protection UVA / UVB composition**

| Component | Action | Content (%) |
|---|---|---|
| Cvclopentasiloxane | Emollient | 8,00 |
| Water | Carrier | qsp 100,00 |
| Glycerin | Moisturizing agent | 5,00 |
| Polygleceryl-4 Isostearate | Emulsifier | 4,00 |
| Phenoxyethanol | Preservative | 1,00 |
| Stearalkonium Hectorite | Rheology modifier | 5,00 |
| Benzotriazolyl tetramehylbutylphenol | Physical filter | 4,00 |
| Methylisothiazolinone | Preservative | 0,07 |
| Candelilla Wax | Consistency agent | 1,50 |
| Titanium Dioxide | Physical filter | 8,00 |
| Zinc Oxide | Physical filter | 18,00 |

### Evaluation of Skin Acceptability in Children

Based on conventional inclusion and exclusion criteria, 32 volunteers of both sexes with age ranging from 3 months to 2 years and 11 months were selected to participate in the evaluation.

The volunteers were initially analyzed by a pediatrician in order to check the inclusion and exclusion criteria and for a general pediatric analysis, the data were completed in the clinical record.

After the analysis, samples of the high protection UVA / UVB composition were distributed together with guidelines defining the use mode. A journal was given so that the volunteer fill out it and give it back at the end of the study.

New pediatric analysis was performed with 21 ± 2 days of study, the data were duly filled in clinic files. The samples of the high protection UVA / UVB composition were weighed at the beginning and the end of study

From the 32 volunteers, 31 completed the study and 01 volunteers did not return for the final analysis due to personal reasons unrelated to the study. The data from volunteers 10 were not considered due to a protocol violation.

From the 30 volunteers considered to the study, no one showed reaction in the skin, under dermatological, ophthalmologic and pediatric monitoring. The final results of the pediatric evaluation are presented in Table 2.

**Table 2: Final data from pediatric clinical analysis.**

| | **Erythema** | **Edema** | **Dryness** | **Total** | **Classification** | **Reaction Extension** |
|---|---|---|---|---|---|---|
| **01** | | | | | | |
| **02** | 0 | 0 | 0 | 0 | NR | - |
| **03** | 0 | 0 | 0 | 0 | NR | - |
| **04** | 0 | 0 | 0 | 0 | NR | - |
| **05** | 0 | 0 | 0 | 0 | NR | - |
| **06** | 0 | 0 | 0 | 0 | NR | - |
| **07** | 0 | 0 | 0 | 0 | NR | - |
| **08** | 0 | 0 | 0 | 0 | NR | - |
| **09** | 0 | 0 | 0 | 0 | NR | - |
| **10** | | | | | | |
| **11** | 0 | 0 | 0 | 0 | NR | - |
| **12** | 0 | 0 | 0 | 0 | NR | - |
| **13** | 0 | 0 | 0 | 0 | NR | - |
| **14** | 0 | 0 | 0 | 0 | NR | - |
| **15** | 0 | 0 | 0 | 0 | NR | - |
| **16** | 0 | 0 | 0 | 0 | NR | - |
| **17** | 0 | 0 | 0 | 0 | NR | - |
| **18** | 0 | 0 | 0 | 0 | NR | - |
| **19** | 0 | 0 | 0 | 0 | NR | - |
| **20** | 0 | 0 | 0 | 0 | NR | - |
| **21** | 0 | 0 | 0 | 0 | NR | - |
| **22** | 0 | 0 | 0 | 0 | NR | - |
| **23** | 0 | 0 | 0 | 0 | NR | - |
| **24** | 0 | 0 | 0 | 0 | NR | - |
| **25** | 0 | 0 | 0 | 0 | NR | - |
| **26** | 0 | 0 | 0 | 0 | NR | - |
| **27** | 0 | 0 | 0 | 0 | NR | - |
| **28** | 0 | 0 | 0 | 0 | NR | - |
| **29** | 0 | 0 | 0 | 0 | NR | - |
| **30** | 0 | 0 | 0 | 0 | NR | - |
| **31** | 0 | 0 | 0 | 0 | NR | - |
| **32** | 0 | 0 | 0 | 0 | NR | - |

wherein "0" means no erythema, no edema and no scaling and "NR" means no reaction.

Table 3 shows the discomfort feelings reported by the volunteers.

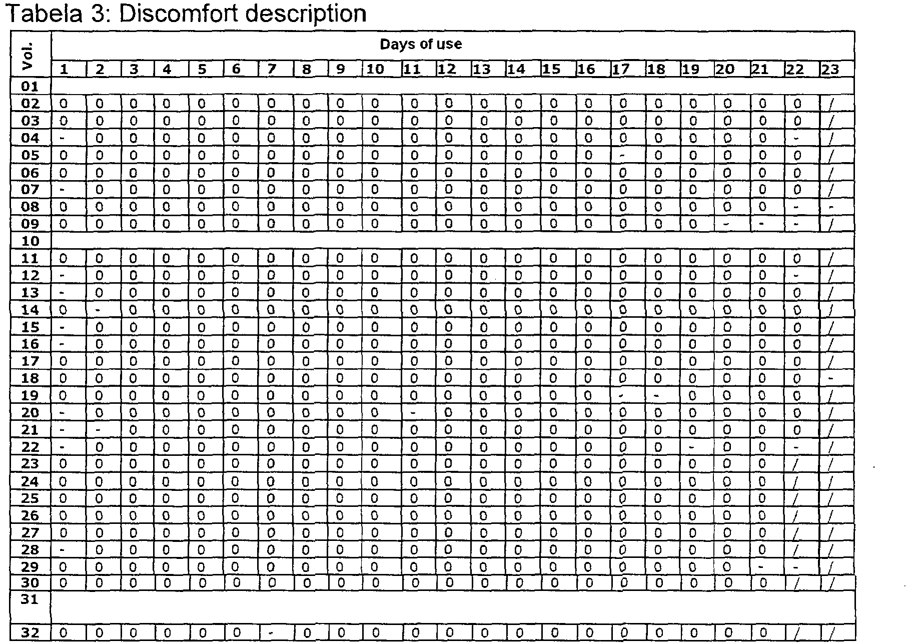

In the above table, "0' means no disconfort feeling; "-" means volunteers did not use the high protection UVA/UVB composition; and "/" means volunteers has already completed the study.

From the above results, it can be concluded that the high protection UVA / UVB of the present invention is safe for human use and any irritation or sensitization reactions causing were caused.

### Evaluation of skin and eye acceptability in adults

Based on the inclusion and exclusion criteria, 32 volunteers of both sexes with age ranging from 18 to 60 years were selected to participate in the evaluation.

The volunteers were initially analyzed by a dermatologist and a oculist in order to check the inclusion and exclusion criteria and for a general dermatologic and ophthalmologic analyses, the data were completed in the clinical record.

After the analyses, samples of the high protection UVA / UVB composition were distributed together with guidelines defining the use mode. A journal was given so that the volunteer fill out it and give it back at the end of the study.

New dermatologic and ophthalmologic analyses were performed with 21 ± 2 days of study, the data were duly filled in clinic records. The samples of the high protection UVA / UVB composition were weighed at the beginning and the end of study

From the 32 volunteers, 31 completed the study, since volunteer 31 did not return for the final analysis due to personal reasons unrelated to the study. The data from volunteer 15 were not considered due to a protocol violation.

Of the 30 volunteers that completed the study showed, no one showed reaction in the skin, ocular or periocular area under dermatological and ophthalmological monitoring. The final results of the dermatologiv evaluation are presented in Table 4.

**Table 4: Final data of dermatologic analysis.**

| | **Erythema** | **Edema** | **Dryness** | **Total** | **Classification** | **Reaction Extension** |
|---|---|---|---|---|---|---|
| **01** | 0 | 0 | 0 | 0 | NR | - |
| **02** | 0 | 0 | 0 | 0 | NR | - |
| **03** | 0 | 0 | 0 | 0 | NR | - |
| **04** | 0 | 0 | 0 | 0 | NR | - |
| **05** | 0 | 0 | 0 | 0 | NR | - |
| **06** | 0 | 0 | 0 | 0 | NR | - |
| **07** | 0 | 0 | 0 | 0 | NR | - |
| **08** | 0 | 0 | 0 | 0 | NR | - |
| **09** | 0 | 0 | 0 | 0 | NR | - |
| **10** | 0 | 0 | 0 | 0 | NR | - |
| **11** | 0 | 0 | 0 | 0 | NR | - |
| **12** | 0 | 0 | 0 | 0 | NR | - |
| **13** | 0 | 0 | 0 | 0 | NR | - |
| **14** | 0 | 0 | 0 | 0 | NR | - |
| **15** | | | | | | |
| **16** | 0 | 0 | 0 | 0 | NR | - |
| **17** | 0 | 0 | 0 | 0 | NR | - |
| **18** | 0 | 0 | 0 | 0 | NR | - |
| **19** | 0 | 0 | 0 | 0 | NR | - |
| **20** | 0 | 0 | 0 | 0 | NR | - |
| **21** | 0 | 0 | 0 | 0 | NR | - |
| **22** | 0 | 0 | 0 | 0 | NR | - |
| **23** | 0 | 0 | 0 | 0 | NR | - |
| **24** | 0 | 0 | 0 | 0 | NR | - |
| **25** | 0 | 0 | 0 | 0 | NR | - |
| **26** | 0 | 0 | 0 | 0 | NR | - |
| **27** | 0 | 0 | 0 | 0 | NR | - |
| **28** | 0 | 0 | 0 | 0 | NR | - |
| **29** | 0 | 0 | 0 | 0 | NR | - |
| **30** | 0 | 0 | 0 | 0 | NR | - |
| **31** | | | | | | |
| **32** | 0 | 0 | 0 | 0 | NR | - |

In the above table, "0" means no erythema, no edema and no scaling and "NR" means no reaction.

Table 5 shows the data from the final ophthalmic analysis of the volunteers.

**Table 5: Data of the final ophthalmic analysis.**

| **N° Vol.** | **Blepharitis** | **Meibomitis** | **Hyperemia** | **Lachrymation** | **Chemosis** | **Cornea** |
|---|---|---|---|---|---|---|
| **01** | 0 | 0 | 0 | 0 | 0 | No modification |
| **02** | 0 | 0 | 0 | 0 | 0 | No modification |
| **03** | 0 | 0 | 0 | 0 | 0 | No modification |
| **04** | 0 | 0 | 0 | 0 | 0 | No modification |
| **05** | 0 | 0 | 0 | 0 | 0 | No modification |
| **06** | 0 | 0 | 0 | 0 | 0 | No modification |
| **07** | 0 | 0 | 0 | 0 | 0 | No modification |
| **08** | 0 | 0 | 0 | 0 | 0 | No modification |
| **09** | 0 | 0 | 0 | 0 | 0 | No modification |
| **10** | 0 | 0 | 0 | 0 | 0 | No modification |
| **11** | 0 | 0 | 0 | 0 | 0 | No modification |
| **12** | 0 | 0 | 0 | 0 | 0 | No modification |
| **13** | 0 | 0 | 0 | 0 | 0 | No modification |
| **14** | 0 | 0 | 0 | 0 | 0 | No modification |
| **15** | | | | | | |
| **16** | 0 | 0 | 0 | 0 | 0 | No modification |
| **17** | 0 | 0 | 0 | 0 | 0 | No modification |
| **18** | 0 | 0 | 0 | 0 | 0 | No modification |
| **19** | 0 | 0 | 0 | 0 | 0 | No modification |
| **20** | 0 | 0 | 0 | 0 | 0 | No modification |
| **21** | 0 | 0 | 0 | 0 | 0 | No modification |
| **22** | 0 | 0 | 0 | 0 | 0 | No modification |
| **23** | 0 | 0 | 0 | 0 | 0 | No modification |
| **24** | 0 | 0 | 0 | 0 | 0 | No modification |
| **25** | 0 | 0 | 0 | 0 | 0 | No modification |
| **26** | 0 | 0 | 0 | 0 | 0 | No modification |
| **27** | 0 | 0 | 0 | 0 | 0 | No modification |
| **28** | 0 | 0 | 0 | 0 | 0 | No modification |
| **29** | 0 | 0 | 0 | 0 | 0 | No modification |
| **30** | 0 | 0 | 0 | 0 | 0 | No modification |
| **31** | | | | | | |
| **32** | 0 | 0 | 0 | 0 | 0 | No modification |

In the above table "0" means lack of reactions.

Table 6 presents the discomfort feelings reported by the volunteers.

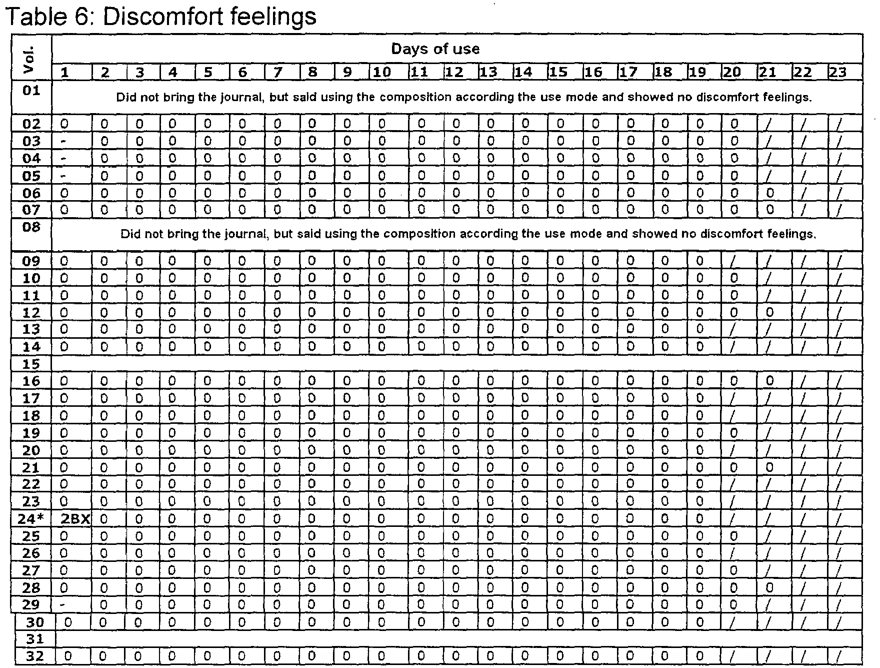

In the above table "0" means no discomfort feeling; "-" means volunteer did not use the high protection UVA/UVB composition; and "/" means volunteer has already completed the study.
* volunteer 24 noted mild rash for about 20 minutes on the first day of composition application. In the remaining days, showed no discomfort feelings and no clinical signs were observed in the final evaluation.

From the above results, it can be concluded that the high protection UVA / UVB of the present invention is safe for human use and any irritation or sensitization reactions causing were caused.

### Evaluation of comedogenic potential

Based on the inclusion and exclusion criteria, 42 volunteers of both sexes with age ranging from 18 to 40 years and phototype (Fitzpatrick) of I to IV were selected to participate in the evaluation.

Samples of the high protection UVA / UVB composition were distributed together with guidelines defining the use mode for 4 weeks. A journal was given so that the volunteer fill out it and give it back at the end of the study.

At the beginning (D0) and at the end (D28) of the study, the volunteers were analyzed by a dermatologist in order to check possible adverse events. The regions analyzed were right or left malar; chin; and right and left forehead.

An exploratory data analysis were performed. The times were compared using a non-parametric Wilcoxon signed rank. The confidence level considered was of 95%. The software used was XSLTAT 2009 and STATA 10.

Of the 42 volunteers, 07 volunteers (003, 004, 010, 011, 020, 021 and 029) were excluded due to a selection failure and 03 volunteers (007, 018 and 030) did not concluded the research due to personal reasons unrelated to the study.

**Table 7 shows the number of injuries by volunteers (the volunteers that did not show no lesions were not included in the table). Table 7: Total number of lesions detected in all areas analyzed by volunteer at D0 and D28.**

| **Volunteer** | **D0** | **D28** | **Δ(D28-D0)** |
|---|---|---|---|
| 001 | 16 | 11 | -5 |
| 002 | 10 | 0 | -10 |
| 005 | 14 | 2 | -12 |
| 006 | 3 | 4 | 1 |
| 008 | 6 | 7 | 1 |
| 009 | 10 | 3 | -7 |
| 012 | 4 | 3 | -1 |
| 013 | 2 | 0 | -2 |
| 014 | 14 | 3 | -11 |
| 015 | 3 | 0 | -3 |
| 016 | 9 | 2 | -7 |
| 017 | 3 | 1 | -2 |
| 019 | 2 | 0 | -2 |
| 022 | 9 | 4 | -5 |
| 023 | 5 | 2 | -3 |
| 024 | 4 | 0 | -4 |
| 025 | 9 | 0 | -9 |
| 026 | 2 | 1 | -1 |
| 027 | 1 | 1 | 0 |
| 028 | 2 | 1 | -1 |
| 031 | 1 | 0 | -1 |
| 032 | 9 | 4 | -5 |
| 033 | 0 | 0 | 0 |
| 034 | 2 | 0 | -2 |
| 035 | 1 | 4 | 3 |
| 036 | 17 | 6 | -11 |
| 037 | 13 | 15 | 2 |
| 038 | 14 | 1 | -13 |
| 039 | 2 | 0 | -2 |
| 040 | 3 | 0 | -3 |
| 041 | 2 | 0 | -2 |
| 042 | 4 | 1 | -3 |
| **Average** | **6,13** | **2,38** | **-3,75** |
| **Median** | 4,0 | 1,0 | -2,5 |
| **Minimum** | 0,0 | 0,0 | -13,0 |
| **Maximium** | 17,0 | 15,0 | 3,0 |
| **EP** | **0,9** | **0,6** | **0,7** |
| IC 95% | 1,8 | 1,2 | 1,5 |
| | **Δ(%) regarding D0** | | **-61,2** |
| | **% volunteers with positive effect** | | **81,3** |
| | **P-Value** | | **< 0,0001***** |

| | | | |
|---|---|---|---|
| *** Significant level of 0.1% | | | |

In view of the above results, it can be concluded that the high protection UVA / UVB composition of the present invention presents no comedogenic potential.

### Evaluation of photoallergy and phototoxicity in the skin

Based on the inclusion and exclusion criteria, 42 volunteers of both sexes with age ranging from 18 to 70 years and phototype (Fitzpatrick) of II and III were selected to participate in the evaluation.

### Photoallergy

A sample was applied always in the same location (right or left dorsal area), properly protected, twice a week for three weeks (total of six applications). The patch test was removed after 24 hours of the application and the area were immediately evaluated and irradiated (12-15 joules) with UVA and UVB light. The adjacent area of application and the point of contact patch with sterile saline solution were irradiated and used as control. After the applications and irradiations, a rest period of at least ten days when no patch and no irradiation were performed occurred.

Then, a patch of the sample was applied in a location where no patch has been placed yet. Tests were removed after 24 hours of application and the test areas were irradiated with UVA light. The adjacent area of application and the point of contact patch with sterile saline solution were irradiated and used as control. The volunteers were instructed to protect from the sun the irradiated skin area. Evaluations were performed after the irradiation and after 24, 48 and 72 hours of the last irradiation.

### Phototoxicity

A sample was applied once, properly protected, the patch test was removed after 24 hours of the application and the area were immediately evaluated and irradiated (12-15 joules) with UVA light. The adjacent area of application and the point of contact patch with sterile saline solution were irradiated and used as control. The volunteers were instructed to protect from the sun the irradiated skin area. Evaluations were performed after the irradiation and after 24, 48 and 72 hours of the last irradiation.

Of the 42 volunteers, 07 volunteers (208, 210, 214, 215, 216, 217 and 232) were excluded in view of a selection failure (SF) and 08 volunteers (201, 209, 213, 223, 224, 237, 239 and 242) withdrew from the study due to personal reasons unrelated to the study. A volunteer (241) showed a reaction in previously sensitized skin, individual pre-disposing, due to skin exposure to the tape, so it was removed from the search. Thus, 26 volunteers completed the study.

No adverse reactions (erythema, edema, papules or vesicles) have been detected in the applications and control areas during, as showed in the following tables.

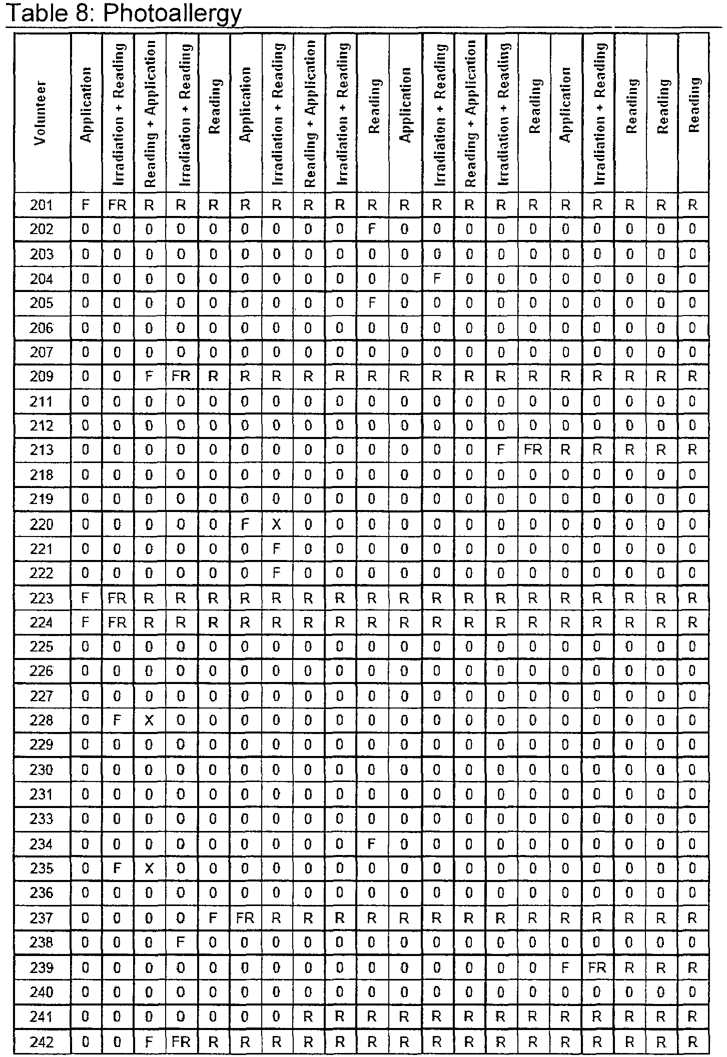

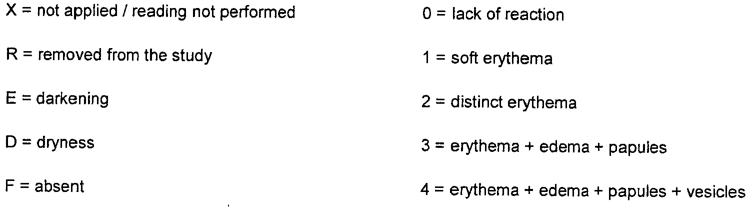

**Tabela 9: Phototoxicity**

| **Application-Reading** | | | | | |
|---|---|---|---|---|---|
| **Volunteer** | **Application** | **Irradiation + Reading** | **Reading 24 h** | **Reading 48 h** | **Reading 72 h** |
| 201 | FR | R | R | R | R |
| 202 | 0 | 0 | 0 | 0 | 0 |
| 203 | 0 | 0 | 0 | 0 | 0 |
| 204 | 0 | 0 | 0 | 0 | 0 |
| 205 | 0 | 0 | 0 | 0 | 0 |
| 206 | 0 | 0 | 0 | 0 | 0 |
| 207 | 0 | 0 | 0 | 0 | 0 |
| 209 | FR | R | R | R | R |
| 211 | 0 | 0 | 0 | 0 | 0 |
| 212 | 0 | 0 | 0 | 0 | 0 |
| 213 | FR | R | R | R | R |
| 218 | 0 | 0 | 0 | 0 | 0 |
| 219 | 0 | 0 | 0 | 0 | 0 |
| 220 | 0 | 0 | 0 | 0 | 0 |
| 221 | 0 | 0 | 0 | 0 | 0 |
| 222 | 0 | 0 | 0 | 0 | 0 |
| 223 | FR | R | R | R | R |
| 224 | FR | R | R | R | R |
| 225 | 0 | 0 | 0 | 0 | 0 |
| 226 | 0 | 0 | 0 | 0 | 0 |
| 227 | 0 | 0 | 0 | 0 | 0 |
| 228 | 0 | 0 | 0 | 0 | 0 |
| 229 | 0 | 0 | 0 | 0 | 0 |
| 230 | 0 | 0 | 0 | 0 | 0 |
| 231 | 0 | 0 | 0 | 0 | 0 |
| 233 | 0 | 0 | 0 | 0 | 0 |
| 234 | 0 | 0 | 0 | 0 | 0 |
| 235 | 0 | 0 | 0 | 0 | a |
| 236 | 0 | 0 | 0 | 0 | 0 |
| 237 | FR | R | R | R | R |
| 238 | 0 | 0 | 0 | 0 | 0 |
| 239 | FR | R | R | R | R |
| 240 | 0 | 0 | 0 | 0 | 0 |
| 241 | R | R | R | R | R |
| 242 | FR | R | R | R | R |

| | | | | | |
|---|---|---|---|---|---|
| X = not applied / reading not performed 0 = lack of reaction R = removed from the study 1 = soft erythema E = darkening 2 = distinct erythema D = dryness 3 = erythema + edema + papules F = absent 4 = erythema + edema + papules + vesicles | | | | | |

From the above results, it can be concluded that the high protection UVA / UVB composition of the present invention does not provokes photoallergy and phototoxicity.

### Evaluation of irritant and allergen potential

Based on the inclusion and exclusion criteria, 72 volunteers of both sexes with age ranging from 18 to 70 years and phototype (Fitzpatrick) of I to IV were selected to participate in the evaluation.

The volunteers were submitted to a general dermatologic analysis by a dermatologist and were received instructions regarding the study.

The study consisted of maximized and controlled applications of the high protection UVA / UVB composition followed by successive readings for six weeks. The composition was applied always in the same dorsal region of the volunteer under semiocclusive dressings. A dressing with saline was used as negative control.

The applications were repeated nine times (D1, D3, D5, D8, D10, D12, D15, D17 and D19) in a period of three consecutive weeks, on alternate days, this period was necessary for a possible induction of immune response or a possible appearance of signs or irritating sensation. The dressing was removed approximately 48 hours after each application, except on weekends wherein the dressings remained in contact with the skin for 72 hours.

To perform all technical evaluations, the excess material were removed with a sterile saline soaked-cotton and waited for a period of at least 30 minutes for the reading. After this induction period, the volunteers began a rest period of two weeks, in which no dressing was applied.

Then, in D36, a single application of the composition was performed at the area where it was performed the induction period and another application on virgin area for a period of 48 hours in order to prove a possible allergic process induced - phase challenge. The dressing was removed after 48 hours of contact (D38) with the skin and the evaluations about 30 minutes and 24 hours after its removal (D39).

A dressing with a saline solution was used as control in all applications of the induction phase and single phase implementation challenge both the virgin area and in the initial area.

Of the 72 volunteers, 04 volunteers (121, 151, 157 e 171) were excluded in view of a selection failure (SF) and 18 volunteers (105, 110, 116, 117, 118, 129, 131, 134, 136, 146, 148, 150, 153, 156, 158, 164, 166 e 169) withdrew from the study due to personal reasons unrelated to the study. Thus, 50 volunteers completed the study.

No adverse reactions (erythema, edema, papules or vesicles) have been detected in the applications and control areas during, as showed in Table 10.

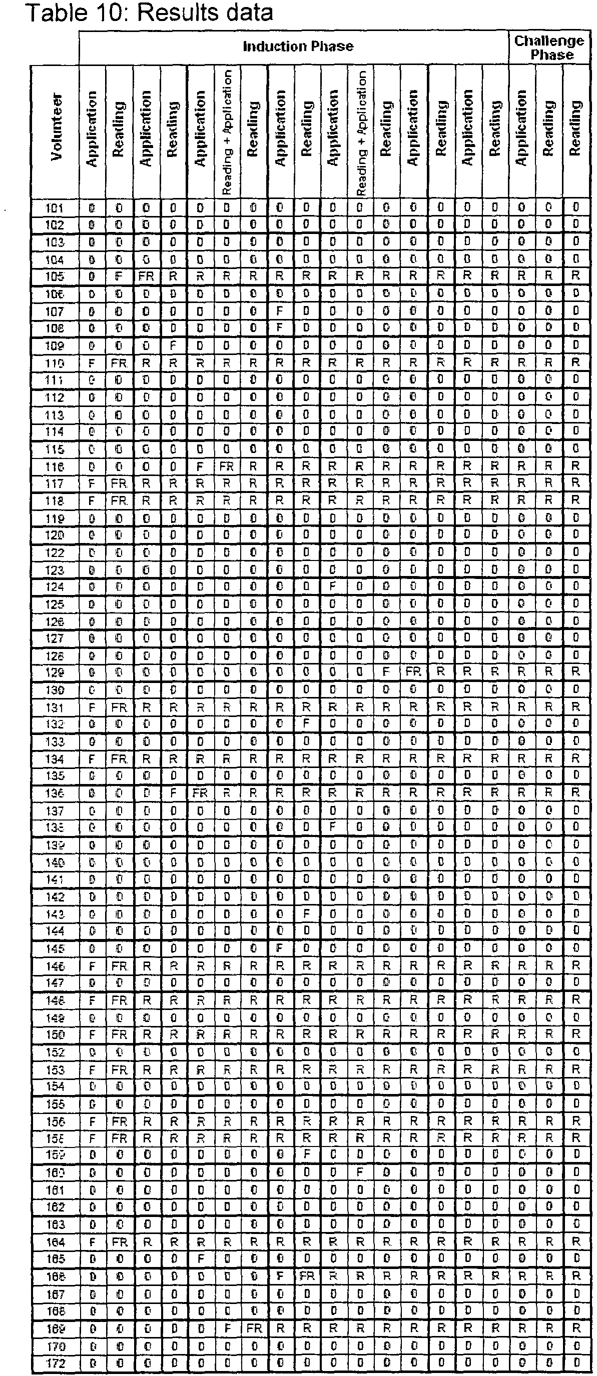

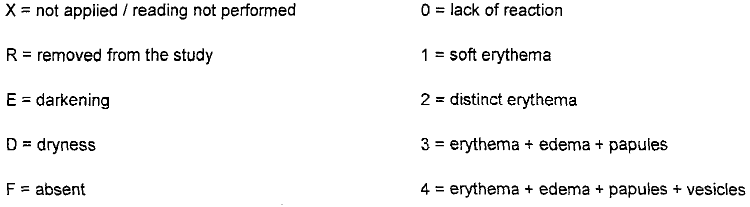

In view of the above results, it can be concluded that the high protection UVA / UVB composition of the present invention does not induces irritation and sensitization process in the skin.

### Evaluation of skin tolerance

Based on the inclusion and exclusion criteria, 10 volunteers of both sexes with age ranging from 18 to 60 years and phototype (Fitzpatrick) of I to IV were selected to participate in the evaluation.

The high protection UVA / UVB composition was applied in the "elbow bending" (in this region, the skin is thin and reactive) twice daily for four consecutive days and once on the fifth day. After the first application of each day, both forearms were flexed on the arm for about 15 minutes. At the end of time, the forearms were extended for about 30 minutes. The area was rinsed with running water.

The clinical examination of the experimental area and a interview with the volunteers were performed by the person responsible by the study. The examination was performed visually under standard "daylight" before application and 45 ± 5 minutes after each application. At the same time, each volunteer was questioned about any possible discomfort feelings. The results are show in Table 11.

**Table 11: Results data**

| **Clinical Manifestations** | **Volunteers Presenting Clinical Manifestations** | **Discomfort Feelings** | **Volunteers Presenting Discomfort Feelings** |
|---|---|---|---|
| None | 0% | None | 0% |

In view of the above results, it can be concluded that the high protection UVA / UVB composition of the present invention has a very good skin compatibility.

### Evaluation of photostability

Small drops of the high protection UVA / UVB composition was applied to the substrate over the entire plate of PMMA - poly(methyl methacrylate). A plate of PMMA with dispersed glycerin was used to record the baseline of the spectrophotometer.

Then the composition drops was quickly spread over the entire surface of the PMMA plate to obtain a visually uniform film. Then a standard drying process consisting in maintaining the composition applied on the substrate for a period of 15 minutes under controlled temperature at 20°C ± 3 °C was performed.

The samples were taken to Suntest CPS+ solar simulator with a fixed irradiance level of 750W/m², a pre-set exposure time and a controlled temperature of the exposing chamber (not more than 40°C).

In Labsphere UV1000S spectrophotometer, absorbance readings were performed at four positions in the sample initially established according to the exposure times of 12, 24, 48 and 96 minutes in Suntest CPS+ solar simulator with an irradiance of 750W/m².

Through the natural radiation conditions, the periods of simulated irradiation of 12, 24, 48 and 96 minutes correspond to 1, 2, 4 and 8 hours of natural sunlight exposure, respectively. The decreasing kinetic in the UV absorption levels were used to evaluate the photostability of the high protection UVA / UVB composition.

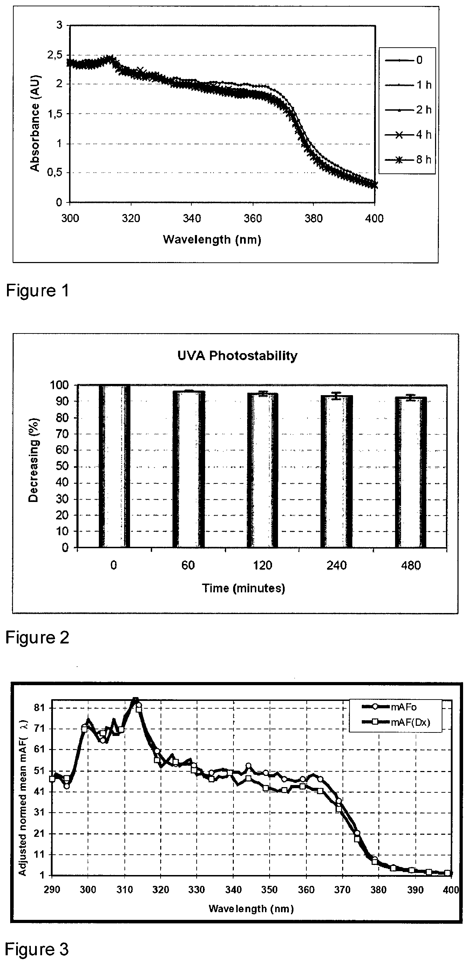

The results of the present study are shown in Figure 1 and Figure 2. Through the analysis thereof, it is observed that the maximum percentage decays obtained (after 8 hours of natural sunlight) were 7.54% in the UVA region, hence the high protection UVA / UVB composition shows high photostability in the UVA region.

### Evaluation of sun protection factor

Based on the inclusion and exclusion criteria, 11 volunteers of both sexes with phototype (Fitzpatrick) of I to III were selected to participate in the evaluation. One volunteer (03) discontinued prior to the completion of the test for personal reasons which were note related to the study.

The area tested was the back between the beltline and the scapulae (shoulder blades) and lateral to midline. Two test site areas were used: one for before water immersion and one for after 360 minutes of water immersion (9 procedures of 30 minutes of immersion in moderately agitated water followed by a 10 minute rest), then the test sites were air dried for 15 minutes without toweling. One additional test site area was used for the P3 control. After applying the composition, a waiting period of 15 to 30 minutes was employed.

A series of UV light exposures was administered to the subsites on each subject with the Modified Solar Ultraviolet Simulator, Model 10S, (the source of illumination for the test site scoring was a warm white fluorescent light which provided a level of 450-550 lux). One series of exposures were administered to the untreated, unprotected skin to determine the MED (minimal erythema dose). The protected test sites were also exposed to UV light. The standard time intervals selected were a geometric series represented by (1.25)n and (1.12)n.

No adverse reactions nor adverse events were observed in any of the subjects, as shown in Table 13. Static SPF (95% Cl) = 65.76 (63.59-67.93) / VWR (very water resistence) SPF (95% Cl) = 60.07 (57.77.-62.37).

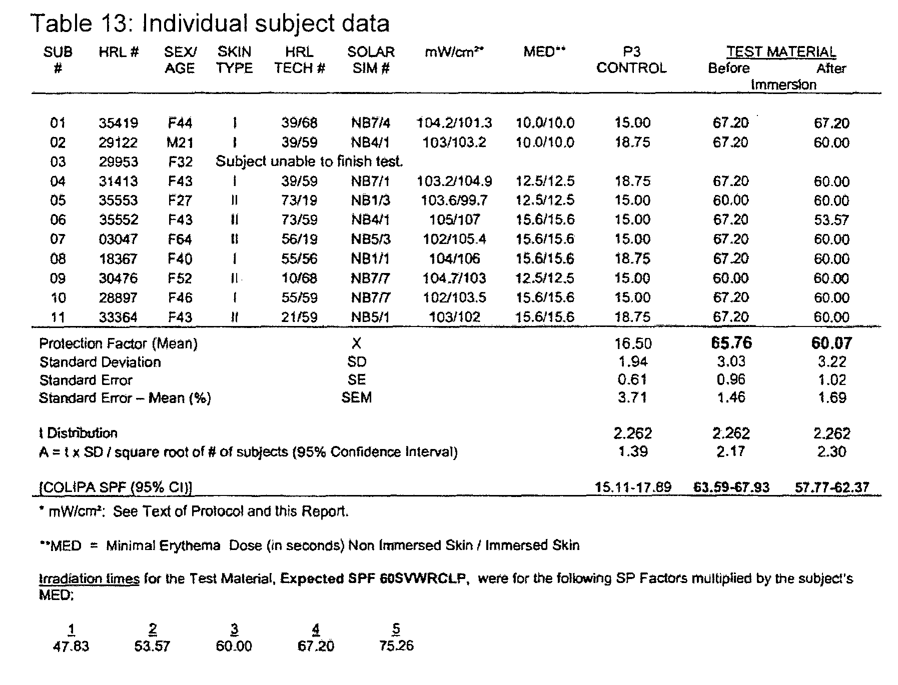

### Evaluation of UVA protection

The present evaluation is based on the measurement of UV transmittance through the sample that is applied to a suitable substrate and with controlled roughness. The composition is exposed to a radiation dosage proportional to the initial UVA Protection Factor (FPUVA₀), calculated by using the transmittance data of a non-exposed sample. The final UVA Protection Factor (FPUVA) is calculated by using the transmittance data adjusted of the sample exposed to UV radiation.

Small drops of the high protection UVA / UVB composition was applied to the substrate over the entire plate of PMMA - poly(methyl methacrylate). A plate of PMMA with dispersed glycerin was used to record the baseline of the spectrophotometer.

Then the composition drops was quickly spread over the entire surface of the PMMA plate to obtain a visually uniform film. Then a standard drying process consisting in maintaining the composition applied on the substrate for a period of 15 minutes under controlled temperature at 20°C ± 3°C was performed.

Each PMMA plate was measured at four different areas to ensure that at least 2 cm² are evaluated. The FPUVA₀ and FPUVA of a plate is calculated by using the absorbance average of all areas of the same PMMA plate. As result acceptance criteria, it was stipulated that if the coefficient of variation for the absorbance between the different areas exceeds 50%, then this PMMA plate must be rejected and a new plate should be prepared.

The FPUVA₀ and FPUVA of the composition is the average of FPUVA₀ and FPUVA of at least three individual plates. As result acceptance criteria, it was stipulated that if the coefficient of variation between FPUVA₀ and FPUVA of individual plates exceeds 20% then new plates should be prepared.

The conditions of the present study were described in Table 12.

**Table 12: Study conditions**

| | |
|---|---|
| SPF in vivo | 65,76 |
| SP-UVAo | 25,33 |
| Adjustment coefficient for SPFo = SPFn | 0,74 |
| Dx (with Do by SP-UVAo unity:) | 30,40 |
| Irradiance (W.m-²) | 750 |
| UV exposure (Suntest) | 22:30 |
| *SP-UVA in vitro 2007* Colipa | 22,62 |

The high protection UVA / UVB composition spectrum before and after radiation exposure are shown in Figure 3. The SPF value of 65.76 (resulted of an *in vivo* test) was used for calculating the UVA protection. Therefore, the composition of the present invention has UVA protection effectiveness, since the average UVA protection values are 22,62.

### Evaluation of stability

Aparência: As amostras submetidas as condições de 45°C, 37°C, 5°C e 25°C por 3 meses não apresentaram variações significativas.

Cor: As amostras submetidas às condições de 45°C, 37°C, 5°C e 25°C por 3 meses não apresentaram variações significativas.

Odor: As amostras submetidas às condições de 45°C, 37°C, 5°C e 25°C por 3 meses não apresentaram variações significativas.

In this study, the high protection UVA / UVB composition was submitted under accelerated aging conditions (45°C, 37°C and 5°C and 25°C for 3 months) in order to evaluate the following parameters:
- Physicochemical Parameter:
   Viscosity: the samples submitted to the accelerated aging conditions kept the viscosity within the specified range.
- Organoleptic parameters:
   Appearance: the samples submitted to the accelerated aging conditions showed no significant variations.
   Color: The samples submitted to the accelerated aging conditions showed no significant variations.
   Odor: Samples submitted to the accelerated aging conditions s showed no significant variations.

## Claims

1. A high protection UVA/UVB cosmetic or pharmaceutical composition, **characterized by** comprising:
(a) 24 to 45.5 % by weight of physical filter selected from the group comprising titanium dioxide particles, zinc oxide particles, a benzotriazole-based compound particles and mixtures thereof;
(b) 6 to 10% by weight of a non-animal hectorite dispersion, and
(c) optionally, 2 to 20% by weight of a sensory modifier selected from the group comprising: silicones, esters, silica and mixtures thereof,
wherein all percentages are based on the total weight of the high protection UVA / UVB composition;
wherein the composition is a topical composition.

2. Composition, according to claim 1, **characterized in that** the content of physical filters is 34% by weight based on the total weight of the high protection UVA / UVB composition.

3. Composition, according to claim 1 or claim 2, **characterized in that** the titanium dioxide particles are present in an amount ranging from 5 to 15%, by weight, based on total weight of the composition.

4. Composition, according to any one of claims 1 to 3, **characterized in that** the zinc oxide particles are present in an amount ranging from 10 to 30% by weight, based on total weight of the composition.

5. Composition, according to any one of claims 1 to 3, **characterized in that** titanium dioxide particles and zinc oxide particles are both present in the composition.

6. Composition, according to any one of claims 1 to 5, **characterized in that** the benzotriazole-based compound is present in an amount ranging from 1 to 10% by weight, based on total weight of the composition.

7. Composition, according to any one of claims 1 to 6, **characterized in that** the benzotriazole-based compound is methylene bis-benzotriazolyl tetramethylbutyl phenol.

8. Composition, according to any one of claims 1 to 7, **characterized in that** any titanium dioxide particles present are dispersed in a cosmetically acceptable lipid carrier, or any zinc oxide particles present are dispersed in a cosmetically acceptable lipid carrier.

9. Composition, according to any one of claims 1 to 8, **characterized in that** the dispersion of non-animal hectorite is present in an amount ranging from 7 to 9% by weight, based on total weight of the composition, for example an amount of 8% by weight, based on total weight of the composition.

10. Composition, according to any one of claims 1 to 9, **characterized in that** the non-animal hectorite dispersion is organically modified with caprylic/capric triglyceride.

11. Composition, according to any one of claims 1 to 10, **characterized in that** the sensory modifier is present in an amount ranging from 5 to 15% by weight, based on total weight of the composition, for example an amount of 10% by weight, based on total weight of the composition.

12. Composition, according to any one of claims 1 to 11, **characterized in that** the sensory modifier is selected from the group consisting of: silicones, esters of short chain, silica and mixtures thereof.

13. Composition, according to claim 12, **characterized in that** the sensory modifier is selected from the group comprising: dimethicone, caprylyl methicone, cyclomethicone, tocopheryl acetate, polysiloxane and mixtures thereof.

14. Composition, according to any one of claims 1 to 13, **characterized in that** the sensory modifier is cyclomethicone, caprylyl methicone or mixtures thereof.

## Patentansprüche

1. Kosmetische oder pharmazeutische Zusammensetzung mit hohem UVA/UVB-Schutz, **dadurch gekennzeichnet, dass** sie umfasst:
(a) 24 bis 45, 5 Gew.-% eines physikalischen Filters, der aus der Gruppe ausgewählt ist, die Titandioxidpartikel, Zinkoxidpartikel, Partikel einer Verbindung auf Benzotriazolbasis und Gemische von diesen umfasst;
(b) 6 bis 10 Gew.-% einer nicht tierischen Hektoritdispersion, und
(c) optional 2 bis 20 Gew.-% eines sensorischen Modifikators, der aus der Gruppe ausgewählt ist, die umfasst: Silikone, Ester, Kieselerde und Gemische von diesen,
wobei alle Prozentangaben auf dem Gesamtgewicht der Zusammensetzung mit hohem UVA/UVB-Schutz beruhen;
wobei es sich bei der Zusammensetzung um eine topische Zusammensetzung handelt.

2. Zusammensetzung nach Anspruch 1,
**dadurch gekennzeichnet, dass** der Gehalt an physikalischen Filtern 34 Gew.-% auf Grundlage des Gesamtgewichts der Zusammensetzung mit hohem UVA/UVB-Schutz beträgt.

3. Zusammensetzung nach Anspruch 1 oder 2, **dadurch gekennzeichnet, dass** die Titandioxidpartikel in einer Menge vorhanden sind, die von 5 bis 15 Gew.-% auf Grundlage des Gesamtgewichts der Zusammensetzung reicht.

4. Zusammensetzung nach einem der Ansprüche 1 bis 3, **dadurch gekennzeichnet, dass** die Zinkoxidpartikel in einer Menge vorhanden sind, die von 10 bis 30 Gew.-% auf Grundlage des Gesamtgewichts der Zusammensetzung reicht.

5. Zusammensetzung nach einem der Ansprüche 1 bis 3, **dadurch gekennzeichnet, dass** Titandioxidpartikel und Zinkoxidpartikel beide in der Zusammensetzung vorhanden sind.

6. Zusammensetzung nach einem der Ansprüche 1 bis 5, **dadurch gekennzeichnet, dass** die Verbindung auf Benzotriazolbasis in einer Menge vorhanden ist, die von 1 bis 10 Gew.-% auf Grundlage des Gesamtgewichts der Zusammensetzung reicht.

7. Zusammensetzung nach einem der Ansprüche 1 bis 6, **dadurch gekennzeichnet, dass** es sich bei der Verbindung auf Benzotriazolbasis um Methylen-bis-benzotriazolyltetramethylbutylphenol handelt.

8. Zusammensetzung nach einem der Ansprüche 1 bis 7, **dadurch gekennzeichnet, dass** jegliche vorhandene Titandioxidpartikel in einem kosmetisch akzeptablen Lipidträger dispergiert sind, oder jegliche vorhandene Zinkoxidpartikel in einem kosmetisch akzeptablen Lipidträger dispergiert sind.

9. Zusammensetzung nach einem der Ansprüche 1 bis 8, **dadurch gekennzeichnet, dass** die Dispersion nicht tierischen Hektorits in einer Menge, die von 7 bis 9 Gew.-% auf Grundlage des Gesamtgewichts der Zusammensetzung reicht, zum Beispiel einer Menge von 8 Gew.-% auf Grundlage des Gesamtgewichts der Zusammensetzung vorhanden ist.

10. Zusammensetzung nach einem der Ansprüche 1 bis 9, **dadurch gekennzeichnet, dass** die nicht tierische Hektoritdispersion mit Capryl/Caprin-Triglycerid organisch modifiziert ist.

11. Zusammensetzung nach einem der Ansprüche 1 bis 10, **dadurch gekennzeichnet, dass** der sensorische Modifikator in einer Menge, die von 5 bis 15 Gew.-% auf Grundlage des Gesamtgewichts der Zusammensetzung reicht, zum Beispiel einer Menge von 10 Gew.-% auf Grundlage des Gesamtgewichts der Zusammensetzung vorhanden ist.

12. Zusammensetzung nach einem der Ansprüche 1 bis 11, **dadurch gekennzeichnet, dass** der sensorische Modifikator aus der Gruppe ausgewählt ist, die besteht aus: Silikonen, kurzkettigen Estern, Kieselerde und Gemischen von diesen.

13. Zusammensetzung nach Anspruch 12, **dadurch gekennzeichnet, dass** der sensorische Modifikator aus der Gruppe ausgewählt ist, die umfasst: Dimethicon, Caprylylmethicon, Cyclomethicon, Tocopherylacetat, Polysiloxan und Gemischen von diesen.

14. Zusammensetzung nach einem der Ansprüche 1 bis 13, **dadurch gekennzeichnet, dass** es sich bei dem sensorischen Modifikator um Cyclomethicon, Caprylylmethicon oder Gemischen von diesen handelt.

## Revendications

1. Composition cosmétique ou pharmaceutique de haute protection contre les UVA/UVB, **caractérisée** comme comprenant :
(a) 24 à 45,5 % en poids de filtre physique choisi dans le groupe comprenant les particules de dioxyde de titane, les particules d'oxyde de zinc, les particules de composé à base de benzotriazole et leurs mélanges ;
(b) 6 à 10 % en poids d'une dispersion d'hectorite non animale, et
(c) éventuellement, 2 à 20 % en poids d'un modificateur sensoriel choisi dans le groupe comprenant : des silicones, des esters, la silice et leurs mélanges,
où tous les pourcentages sont basés sur le poids total de la composition de haute protection contre les UVA/UVB ;
où la composition est une composition topique.

2. Composition selon la revendication 1, **caractérisée en ce que** la teneur en filtres physiques est de 34 % en poids sur la base du poids total de la composition de haute protection contre les UVA/UVB.

3. Composition selon la revendication 1 ou la revendication 2, **caractérisée en ce que** les particules de dioxyde de titane sont présentes en une quantité se situant dans la plage allant de 5 à 15 % en poids, sur la base du poids total de la composition.

4. Composition selon l'une quelconque des revendications 1 à 3, **caractérisée en ce que** les particules d'oxyde de zinc sont présentes en une quantité se situant dans la plage allant de 10 à 30 % en poids, sur la base du poids total de la composition.

5. Composition selon l'une quelconque des revendications 1 à 3, **caractérisée en ce que** les particules de dioxyde de titane et les particules d'oxyde de zinc sont toutes les deux présentes dans la composition.

6. Composition selon l'une quelconque des revendications 1 à 5, **caractérisée en ce que** le composé à base de benzotriazole est présent en une quantité se situant dans la plage allant de 1 à 10 % en poids, sur la base du poids total de la composition.

7. Composition selon l'une quelconque des revendications 1 à 6, **caractérisée en ce que** le composé à base de benzotriazole est le méthylène-bis-benzotriazolyl-tétraméthylbutylphénol.

8. Composition selon l'une quelconque des revendications 1 à 7, **caractérisée en ce que** toutes les particules de dioxyde de titane présentes sont dispersées dans un véhicule lipidique cosmétiquement acceptable, ou toutes les particules d'oxyde de zinc présentes sont dispersées dans un véhicule lipidique cosmétiquement acceptable.

9. Composition selon l'une quelconque des revendications 1 à 8, **caractérisée en ce que** la dispersion d'hectorite non animale est présente en une quantité se situant dans la plage allant de 7 à 9 % en poids, sur la base du poids total de la composition, par exemple de 8 % en poids, sur la base du poids total de la composition.

10. Composition selon l'une quelconque des revendications 1 à 9, **caractérisée en ce que** la dispersion d'hectorite non animale est modifiée organiquement avec un triglycéride caprylique/caprique.

11. Composition selon l'une quelconque des revendications 1 à 10, **caractérisée en ce que** le modificateur sensoriel est présent en une quantité se situant dans la plage allant de 5 à 15 % en poids, sur la base du poids total de la composition, par exemple une quantité de 10 % en poids, sur la base du poids total de la composition.

12. Composition selon l'une quelconque des revendications 1 à 11, **caractérisée en ce que** le modificateur sensoriel est choisi dans le groupe constitué par : des silicones, des esters de chaîne courte, la silice et leurs mélanges.

13. Composition selon la revendication 12, **caractérisée en ce que** le modificateur sensoriel est choisi dans le groupe comprenant : la diméthicone, la caprylylméthicone, la cyclométhicone, l'acétate de tocophéryle, le polysiloxane et leurs mélanges.

14. Composition selon l'une quelconque des revendications 1 à 13, **caractérisée en ce que** le modificateur sensoriel est la cyclométhicone, la caprylylméthicone ou leurs mélanges.
